Europäisches Patentamt

European Patent Office

Office européen des brevets

(11) Numéro de publication: **0 256 373**
**A1**

## (12) DEMANDE DE BREVET EUROPEEN

(21) Numéro de dépôt: **87110963.3**

(22) Date de dépôt: **29.07.87**

(51) Int. Cl.⁴ **G01N 1/28** , **F26B 5/06** ,
**A61K 9/48**

(30) Priorité: **11.08.86 FR 8611675**

(43) Date de publication de la demande:
**24.02.88 Bulletin 88/08**

(84) Etats contractants désignés:
**CH DE FR GB LI SE**

(71) Demandeur: **Acepsa Analyses, Conseils et Production SA**

**CH-1041 Oulens(CH)**

(72) Inventeur: **Maire, Nicolas**
**La Laiterie**
**CH-1041 Oulens(CH)**

(74) Mandataire: **Caron, Gérard et al**
**ICB Ingénieurs Conseils en Brevets SA**
**Passage Max. Meuron 6**
**CH-2001 Neuchâtel(CH)**

(54) **Procédé de fabrication d'un élément comportant une quantité déterminée d'une substance donnée et élément obtenu par ce procédé.**

(57) Procédé de fabrication d'un élément comportant une quantité déterminée d'une substance donnée à l'état solide.

Le procédé consiste à introduire dans une capsule (10a), sans la remplir complètement, un volume déterminé d'une solution (11) de la substance en concentration déterminée, ladite capsule (10a) ayant la propriété de se désagréger au contact d'un réactif approprié et/ou sous l'effet d'une action mécanique donnée, à congeler ladite solution (11), à obturer la capsule (10a) par un bouchon poreux (14, 17) déposé sur la solution congelée, à éliminer le solvant de ladite solution (11) par lyophilisation, puis à fermer la capsule (10a).

Application à la réalisation d'étalons d'adénines nucléotides.

Fig. 1a

Fig. 1f

Fig. 1f'

## PROCEDE DE FABRICATION D'UN ELEMENT COMPORTANT UNE QUANTITE DETERMINEE D'UNE SUBSTANCE DONNEE ET ELEMENT OBTENU PAR CE PROCEDE

La présente invention concerne un procédé de fabrication d'un élément comportant une quantité déterminée d'une substance donnée, à l'état solide. L'invention concerne également l'élément obtenu par ce procédé.

D'une manière générale, ce type d'élément est utile pour de nombreuses applications, particulièrement lorsqu'il est nécessaire d'administrer, de dispenser ou d'utiliser de toute autre manière une substance chimique en quantité unitaire prédéterminée, généralement très faible.

C'est le cas, par exemple, pour certains produits chimiques qui sont utilisés en suspension ou en solution mais sont difficiles ou dangereux à transporter ou stocker sous une telle forme. Si l'on dispose alors d'une unité renfermant une quantité donnée de ce produit à l'état stabilisé (c'est-à-dire à l'état solide, en l'absence de solvant), il est facile de recréer, le moment venu, une composition standardisée utilisable notamment en analyse chimique ou pour l'étalonnage d'appareils de mesure.

Ce type d'élément trouve également une application intéressante pour des produits permettant une réaction spécifique (réactifs, catalyseurs, ...), qui doivent être conservés à l'état stabilisé et que l'on ajoute à un échantillon de substance pour en déterminer la teneur en un composant particulier.

Une autre application particulièrement intéressante de ces éléments réside dans l'étalonnage d'un appareil automatique d'extraction et de mesure de la teneur en ATP (adénosine-triphosphate). Ce mononucléotide du métabolisme, universellement représenté dans tous les organismes vivants, assure la transmission ou la mise en réserve d'énergie dans la plupart des réactions biochimiques se déroulant au sein des cellules vivantes (respiration, fermentation, photosynthèse, etc...). L'ATP se trouve donc au centre des échanges énergétiques propres à la vie et permet de doser ces échanges. Dans un milieu naturel, la teneur en ATP donne ainsi une image de l'activité biologique du milieu.

Le dosage de l'ATP dans un milieu peut être avantageusement effectué selon la méthode proposée dans l'article "Extraction de l'adénosine-triphosphate dans les sols: une nouvelle méthode de calcul des pertes en ATP" (N. Maire, Soil Biol. Biochem. Vol.16, No 4, p 361 - 366, 1984).

La méthode comporte les deux phases principales suivantes:

-extraction de la molécule par rupture des parois cellulaires;

-mesure photométrique de la molécule par une réaction enzymatique de bioluminescence, l'intensité de la lumière émise étant directement proportionnelle à la concentration d'ATP.

Cette mesure photométrique nécessite, d'une part, un étalonnage du photomètre lui-même à l'aide d'une gamme étalon d'unités solides d'ATP et, d'autre part, un étalonnage interne du milieu à analyser par adjonction d'une unité solide d'ATP.

Dans toutes les applications précitées, l'unité solide de substance chimique doit être contenue dans un emballage qui a pour fonction, dans un premier temps, d'isoler la substance du milieu à analyser (échantillon) dans lequel elle se trouve, puis de se désintégrer à un moment défini par l'utilisateur au contact d'un agent chimique et/ou sous l'effet d'une contrainte mécanique donnée.

La présente invention a pour but de fournir un élément dans lequel une unité solide de substance chimique est enfermée à l'intérieur d'un emballage répondant aux exigences ci-dessus.

De façon plus précise, l'invention concerne un procédé de fabrication d'un élément comportant une quantité déterminée d'une substance donnée, à l'état solide, caractérisé en ce qu'il consiste à:

-introduire dans une capsule un volume déterminé d'une solution de ladite substance en concentration déterminée, ladite capsule ayant la propriété de se désagréger au contact d'un réactif approprié et/ou sous l'effet d'une action mécanique donnée;

-solidifier ladite solution par abaissement de sa température,

-éliminer le solvant de ladite solution par séchage sous vide; et

-fermer la capsule.

D'autres caractéristiques de l'invention ressortiront de la description qui va suivre, faite en regard des dessins annexés qui illustrent les différentes étapes de deux modes d'exécution du procédé.

En ce qui concerne tout d'abord le premier mode d'exécution et ainsi que le montre la figure 1a, on utilise comme emballage une capsule 1O du type utilisé pour les gélules médicales. Cette capsule, réalisée en deux parties 1Oa - 1Ob qui s'emboîtent l'une dans l'autre par leur extrémité ouverte, doit pouvoir se désintégrer au contact d'un réactif chimique et/ou sous l'effet d'une action mécanique donnée. Pour permettre cette désintégration, la capsule est avantageusement en gélatine, agar-agar, amidon, cellulose, polystyrène ou toute autre matière synthétique friable. Afin de fixer les idées, le volume de la partie 1Oa est de 0,3 ml.

La première étape du procédé, illustrée par la figure 1a, consiste à verser dans la capsule 10a un volume précis d'une solution 11, en concentration bien déterminée, de la substance chimique dont il s'agit de préparer une unité solide. Pour des raisons qui apparaîtront plus loin, la solution 11 ne doit pas remplir la totalité du volume utilisable de la capsule. A titre indicatif, la solution occupe seulement les 2/3 de ce volume, soit 0,2 ml dans l'exemple donné.

Typiquement, lorsque la substance en question est de l'ATP, la solution utilisée est une solution aqueuse de cette substance. Ainsi, lorsque sa concentration est de $10^{-4}$M et que le volume introduit dans la capsule est de 0,2 ml, cela correspond à une unité de $2 \cdot 10^{-8}$ mole d'ATP.

Il est avantageux, dans le cas de l'ATP, d'utiliser comme solvant de l'eau déminéralisée et stérilisée contenant 0,1 % d'azide de sodium ($NaN_3$) agissant en tant qu'agent biocide.

En variante de ce premier mode d'exécution du procédé, de l'agar-agar peut être ajouté au solvant. La solution ainsi obtenue se gélifiant au-dessous de 45°C, il est donc nécessaire, pour réaliser la mise en solution de l'ATP et le remplissage de la gélule, de travailler à une température supérieure à cette valeur.

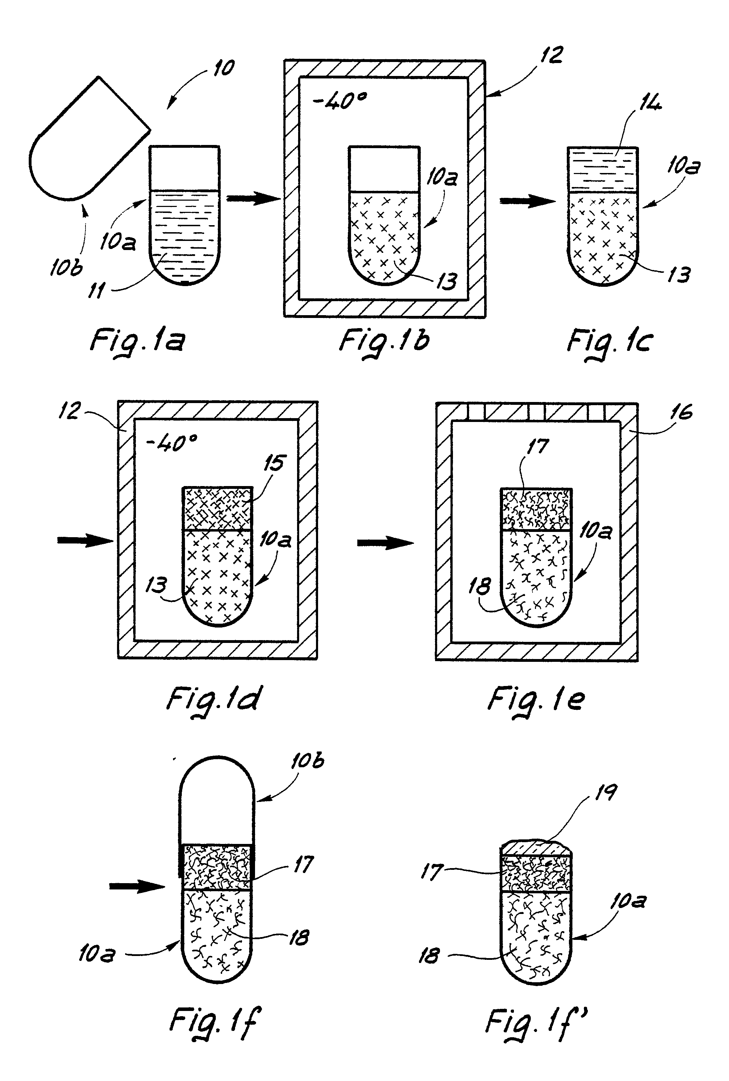

La deuxième étape du procédé, illustrée par la figure 1b, consiste à placer la capsule 10a dans une enceinte réfrigérante 12 afin de congeler la solution 11, désignée alors par la référence 13. Typiquement, la température de l'enceinte est de -40°C.

Après congélation totale de la solution, comme le montre la figure 1c, une seconde solution 14, destinée à former un bouchon dont le rôle apparaîtra plus loin, est introduite dans la partie supérieure de la capsule 10a. Dans l'exemple choisi, cette solution occupe un volume de 0,1 ml, de sorte que la capsule 10a se trouve alors totalement remplie.

La seconde solution 14 est avantageusement formée à partir d'une solution aqueuse à 1,5 % d'agar-agar contenant 0,1 % d'azide de sodium comme agent biocide. Cette solution est préalablement stérilisée par autoclavage pendant 15 minutes à une température de 121°C. Elle est ensuite refroidie à 45°C environ, température à laquelle elle est introduite dans la capsule sur la solution congelée 13. Comme l'illustre la figure 1d, la capsule 10a est alors immédiatement replacée dans l'enceinte 12 à -40°C où la solidification de la solution 14 provoque la formation d'un bouchon 15.

Il faut noter que de la gélatine peut être utilisée en lieu et place d'agar-agar.

L'étape suivante du procédé qui tire profit de la congélation ainsi obtenue et qui est représentée sur la figure 1e, est la sublimation des solvants dans un lyophilisateur 16. Typiquement, cette opération de séchage est effectuée pendant 12 heures pendant lesquelles le solvant du bouchon 15 est éliminé en premier et laisse alors subsister une structure poreuse d'agar-agar ou de gélatine. Cette structure constitue un bouchon poreux 17 formant une barrière qui permet l'élimination du solvant de la première solution 13 mais s oppose au passage de la substance qu'elle contient. Celle-ci se retrouve finalement seule, à l'état solide, désignée par la référence 18, emprisonnée sous le bouchon poreux 17.

Il est à noter que l'opération de lyophilisation qui implique nécessairement une congélation suivie d'un séchage sous vide peut être remplacée par un simple séchage sous vide au cas où la solution 13 se solidifie à température ambiante, par exemple, comme on le verra par la suite.

Il ne reste plus alors, comme le montre la figure 1f, qu'à refermer la capsule en disposant sur la partie remplie 10a la partie 10b formant couvercle.

En variante, le couvercle 10b peut être remplacé par un simple bouchon de cire ou de paraffine 19 déposé sur le bouchon poreux 17, comme représenté sur la figure 1f'.

Ainsi donc est obtenu un élément comportant une capsule 10, soit en deux parties 10a - 10b emboîtées l'une dans l'autre (figure 1f), soit en une seule partie 10a obturée par un bouchon de cire ou de paraffine 19 (forme 1f') et, enfermés dans cette capsule, une substance chimique 18 à l'état solide et un bouchon poreux 17 disposé sur celle-ci.

Bien entendu, et comme déjà mentionné, l'ATP n'est pas la seule substance qui peut être mise en capsule selon le procédé de l'invention. Des éléments peuvent être réalisés, par exemple, avec l'adénosine-diphosphate (ADP) et l'adénosine-monophosphate (AMP) qui constituent avec l'ATP les trois adénines nucléotides, ou avec l'apyrase, la pyruvate-kinase (PK), la phosphoenolpyruvate (PEP), la myokinase ... ou des mélanges de ces substances.

L'opération de remplissage illustrée à la figure 1a peut comprendre l'introduction d'une certaine quantité d'une substance de lestage qui permet, lorsque la capsule est utilisée ultérieurement en la soumettant à un broyage dans une solution d'utilisation, de la maintenir au fond du récipient d'analyse. Cette substance de lestage peut être une poudre minérale telle que du calcaire ou de la silice. Bien entendu, le volume et la concentration de la solution 11 doivent alors être adaptés en conséquence, de manière à ce que le mélange obtenu occupe au maximum les deux-tiers du volume utile de la capsule.

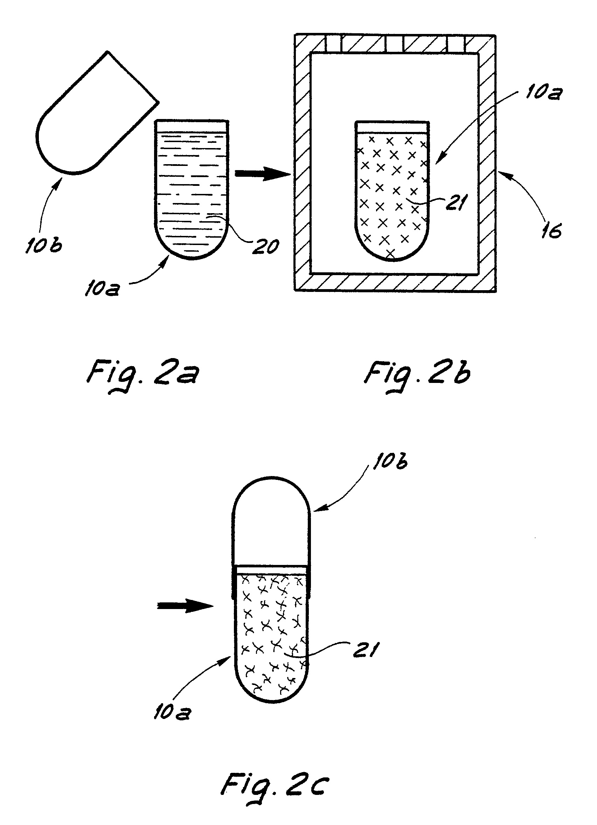

Les figures 2a à 2c illustrent un second mode d'exécution de l'invention qui est plus simple mais qui conduit à sacrifier légèrement la précision de la détermination de la substance dans le produit final. Dans ce second mode d'exécution, on met à profit la température de gélification de l'agar-agar qui est ajouté au solvant utilisé pour préparer la première solution. En effet, comme représenté à la figure 2a, une solution 20 avantageusement du même type que précédemment décrit, mais contenant nécessairement de l'agar-agar est introduite dans la capsule 10a en la remplissant pratiquement jusqu'au bord et en travaillant à une température supérieure à 45°C. Puis, la capsule remplie est ramenée à température ambiante. On peut alors placer la capsule 10a dans une enceinte de séchage sous vide 16 (figure 2b) pour éliminer le solvant de la solution 20 pour former la substance à l'état solide 21, qui se trouve ainsi emprisonnée directement dans la structure poreuse formée par l'agar-agar, après quoi la capsule 10a est munie de son couvercle 10b.

Ce deuxième mode d'exécution qui ramène le procédé à quelques étapes très simples peut être complété éventuellement par une étape consistant à créer le bouchon 14 (figure 1c) cas auquel, bien entendu, la capsule 10a ne doit pas être remplie jusqu'à son bord. Cette capsule peut alors être soumise, soit à un simple séchage sous vide, soit à une lyophilisation complète impliquant une congélation préalable (figure 1d) puis le traitement dans l'enceinte 16 (figure 1e), comme décrit à propos du premier mode d'exécution du procédé suivant l'invention.

**Revendications**

1. Procédé de fabrication d'un élément comportant une quantité déterminée d'une substance donnée, à l'état solide, caractérisé en ce qu'il consiste à:

-introduire dans une capsule (10a) un volume déterminé d'une première solution (11; 20) de ladite substance en concentration déterminée, ladite capsule ayant la propriété de se désagréger au contact d'un réactif approprié et/ou sous l'effet d'une action mécanique donnée;

-solidifier ladite première solution par abaissement de sa température (13; 21),

-éliminer le solvant de ladite première solution par séchage sous vide; et

-fermer la capsule.

2. Procédé selon la revendication 1, caractérisé en ce qu'il consiste en outre à obturer la capsule (10a) par un bouchon poreux (14) déposé sur la première solution solidifié (13), préalablement à l'opération d'élimination du solvant de cette première solution.

3. Procédé selon l'une quelconque des revendications 1 et 2, caractérisé en ce que ladite substance comprend une adénine nuclotide, du groupe formé par l'adénosine-triphosphate, l'adénosine-diphosphate et l'adénosine-monophosphate.

4. Procédé selon l'une quelconque des revendications 1 à 3, caractérisé en ce que le solvant est de l'eau déminéralisée et stérilisée.

5. Procédé selon l'une quelconque des revendications 1 à 3, caractérisé en ce que le solvant de la première solution est elle-même une solution d'agar-agar.

6. Procédé selon l'une quelconque des revendications 1 à 5, caractérisé en ce que ladite opération de solidification par abaissement de la température est une opération de congélation.

7. Procédé selon l'une quelconque des revendications 1 à 6, caractérisé en ce que l'opération de séchage sous vide est une opération de lyophilisation impliquant une opération de congélation préalable au séchage.

8. Procédé selon l'une quelconque des revendications 2 à 7, caractérisé en ce que le bouchon poreux (14) est formé à partir d'une seconde solution d'agar-agar ou de gélatine versée sur la première solution solidifiée (13), le solvant de cette seconde solution d'agar-agar ou de gélatine étant éliminé lors du séchage sous vide pour laisser subsister une structure d'agar-agar ou de gélatine (17) formant ledit bouchon poreux.

9. Procédé selon la revendication 8, caractérisé en ce que la seconde solution utilisée pour former le bouchon poreux (17) est une solution aqueuse à 1,5 % d'agar-agar ou de gélatine.

10. Procédé selon l'une quelconque des revendications 1 à 9, caractérisé en ce que le solvant de ladite première solution contient un agent biocide.

11. Procédé selon l'une quelconque des revendication 8 à 10, caractérisé en ce que le solvant de ladite seconde solution contient un agent biocide.

12. Procédé selon l'une quelconque des revendications 1 à 11, caractérisé en ce qu'il consiste en outre à introduire dans la capsule une substance de lestage telle que du calcaire ou de la silice.

13. Elément obtenu par le procédé selon l'une quelconque des revendications précédentes, caractérisé en ce qu'il comporte une capsule (10a) capable de se désintégrer au contact d'un réactif chimique et/ou sous l'effet d'une action mécanique et, à l'intérieur de cette capsule une quantité déterminée d'une substance chimique à l'état solide (18; 21).

14. Elément selon la revendication 13, caractérisé en ce qu'un bouchon poreux (17) est disposé sur ladite substance.

15. Elément selon l'une quelconque des revendications 13 et 14, caractérisé en ce que ladite capsule (10a) est réalisée en l'un des produits du groupe comprenant la gélatine, l'agar-agar, l'amidon, la cellulose et le polystyrène.

16. Elément selon l'une quelconque des revendications 13 à 15, caractérisé en ce que la capsule est réalisée en deux parties (10a, 10b) s'emboîtant l'une dans l'autre, à l'instar des capsules utilisées pour les gélules médicales.

17. Elément selon l'une des revendications 13 à 15, caractérisé en ce que la capsule (10a) est obturée par un bouchon de cire ou de paraffine (19).

Fig.1a  Fig.1b  Fig.1c

Fig.1d  Fig.1e

Fig.1f  Fig.1f'

Fig. 2a

Fig. 2b

Fig. 2c

## DOCUMENTS CONSIDERES COMME PERTINENTS

| Categorie | Citation du document avec indication, en cas de besoin, des parties pertinentes | Revendication concernee | CLASSEMENT DE LA DEMANDE (Int Ci 4) |
|---|---|---|---|
| Y | GB-A- 825 480 (MED FABRIK CHEMISCH- PHARMAZEUTISCHER PRÄPARATE J.C. PFLÜGER) * Page 2, lignes 112-127; page 3, lignes 2-27; page 3, lignes 63-93; page 3, lignes 102-113; page 4, lignes 1-9; revendications 1,3,4,12 * | 1,6,7, 13,15 | G 01 N 1/28  F 26 B 5/06  A 61 K 9/48 |
| Y | US-A-4 305 502 (G.K.E. GREGORY et al.) * Colonne 2, ligne 45 - colonne 3, ligne 2; colonne 3, lignes 21-35; colonne 3, lignes 52 - colonne 4, ligne 10; colonne 4, lignes 25-34; colonne 5, lignes 12-30; figure 3; revendications 1,2,7 * | 1,6,7, 13,15 | |
| A | | 4 | |
| A | EP-A-0 084 705 (JOHN WYETH & BROTHER LTD) * Page 3, ligne 21 - page 4, ligne 4; page 4, lignes 17-27; page 5, lignes 16-21; page 6, lignes 22-27; page 7, lignes 17-26; page 8, lignes 8-14,23-29; page 10, lignes 8-14; page 13, example b; page 14, example c; revendications 1,2,7 * | 1,6,7 | |

DOMAINES TECHNIQUES RECHERCHES (Int. Cl.4)

G 01 N 1
F 26 B 5
A 61 K 9

--- -/-

Le présent rapport de recherche a été établi pour toutes les revendications

| Lieu de la recherche | Date d'achèvement de la recherche | Examinateur |
|---|---|---|
| LA HAYE | 02-11-1987 | FORMBY N.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié a la date de dépôt ou après cette date
D : cité dans la demande
L : cité pour d'autres raisons

& : membre de la même famille, document correspondant

OEB Form 1503 03 82

## DOCUMENTS CONSIDERES COMME PERTINENTS

Page 2

| Categorie | Citation du document avec indication, en cas de besoin, des parties pertinentes | Revendication concernee | CLASSEMENT DE LA DEMANDE (Int. Cl. 4) |
|---|---|---|---|
| A | BE-A- 699 719 (SOCIETE DES PRODUITS NESTLE SA) <br> * Page 1, ligne 10 - page 2, ligne 18; page 3, lignes 2-13; page 4, lignes 8-24; page 5, lignes 9-26; revendications 1,2,6,9,18; page 6, lignes 4-22 * <br><br> --- | 1,6 | |
| A | DE-A-2 729 068 (R. LIEDTKE) <br> * Page 3, ligne 24 - page 4, ligne 21; page 5, lignes 1-12; figure 1; revendication 1 * <br><br> ----- | 15,16 | |

DOMAINES TECHNIQUES
RECHERCHES (Int. Cl.4)

Le présent rapport de recherche a été établi pour toutes les revendications

| Lieu de la recherche <br> LA HAYE | Date d'achèvement de la recherche <br> 02-11-1987 | Examinateur <br> FORMBY N.M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82